(19)

Europäisches
Patentamt
European
Patent Office
Office européen
des brevets

(11) **EP 2 023 256 A1**

(12) **EUROPEAN PATENT APPLICATION**

(43) Date of publication:
**11.02.2009 Bulletin 2009/07**

(51) Int Cl.:
*G06F 19/00* $^{(2006.01)}$     *A61B 5/00* $^{(2006.01)}$
*A61M 5/172* $^{(2006.01)}$     *G01N 33/487* $^{(2006.01)}$

(21) Application number: **07113689.9**

(22) Date of filing: **02.08.2007**

| | |
|---|---|
| (84) Designated Contracting States:<br>**AT BE BG CH CY CZ DE DK EE ES FI FR GB GR HU IE IS IT LI LT LU LV MC MT NL PL PT RO SE SI SK TR**<br>Designated Extension States:<br>**AL BA HR MK RS** | (71) Applicant: **NOVO NORDISK A/S**<br>**2880 Bagsvaerd (DK)**<br><br>(72) Inventor: **The designation of the inventor has not yet been filed** |

(54) **Drug administration monitoring**

(57)    Disclosed herein is an apparatus for monitoring administration of a medication to a user. The apparatus comprising processing means adapted to obtain input values of at least a physiological parameter of the user measured prior to and after intake of a meal by the user, and of at least a dose of medication administered to the user; determine, from at least the obtained input values, an estimate of a nutritional parameter of the meal; and to generate an output indicative of the determined estimate.

Fig. 3

**EP 2 023 256 A1**

**Description**

TECHNICAL FIELD

The present invention relates generally to systems for managing medical therapy. More specifically, the invention relates to the monitoring of the administration/de/ivery of a medication to a user.

BACKGROUND OF THE INVENTION

When a person has a condition which requires self-medication, it is often desirable that the person is able to determine the dose to take under the current circumstances. This is, e.g., the case if a person has insulin-dependent diabetes. Accordingly, in the present description reference is mostly made to the treatment of diabetes by injection of insulin. However, other uses of the apparatus and method described herein are possible.

Diabetes mellitus is the common name for at least two different diseases, one characterised by immune system mediated specific pancreatic beta cell destruction (insulin dependent diabetes mellitus (IDDM) or type 1 diabetes), and another characterised by decreased insulin sensitivity (insulin resistance) and/or a functional defect in beta cell function (non-insulin dependent diabetes mellitus (NIDDM) or type 2 diabetes).

The principal treatment of type 1 diabetes includes the substitution of the missing insulin secretion, whereas treatment of type 2 is more complicated. More specifically, in early stages of type 2 diabetes treatment a number of different types of drugs can be used, e.g. drugs which increase insulin sensitivity (ciglitazones), decrease hepatic glucose output (e.g. metformin), or reduce glucose uptake from the gut (alfa glucosidase inhibitors), as well as drugs which stimulate beta cell activity (e.g. sulfonylurea/meglitinides).

However, the above-described deterioration is reflected in the fact that beta cell stimulators will eventually fail to stimulate the cell, and the person has to be treated with insulin, either as mono therapy, or in combination with oral medication in order to improve glucose control.

Currently, there are two principal modes of daily insulin therapy, one including syringes and insulin injection pens and the other including infusion pump. Syringes and insulin injection pens are simple to use and are relatively low in cost, and they typically involve a number of injections during the day, e.g. 3-4 times or more per day. Typically, the injections are administered in connection with a meal.

In order to maintain a proper dosing of insulin, whether administered with an insulin injecting pen or with an infusion pump, most persons having diabetes are required more or less frequently to monitor the blood glucose in order to maintain an acceptable treatment with insulin. Monitoring of blood glucose may be performed by means of glucose meters which typically rely on small blood samples taken with a lancet. Conventional glucose monitoring systems typically take readings as directed by the user and may provide a warning if a reading is deemed at an unsafe level (e.g., a hyper- or hypoglycaemic condition).

In the case of self-medication of insulin or other medicaments, the person may need to take regular "background" doses of medication as well as bolus doses immediately prior to the consumption of a meal in order to compensate for the food intake during the meal. The correct dose, in particular in the case of a bolus dose, depends on many different factors, and it is therefore difficult for the person to readily estimate or calculate a correct dose.

The above difficulty of correct calculation of the dose will often result in the person taking an average dose without taking any of the specific circumstances relating to the current situation into account. This is a disadvantage because such an average dose will very seldom correspond to the exact need for medication for that particular person at that particular time. Even if the person takes the current situation into account, e.g. by estimating the relevant properties, e.g. the nutritional composition, of the consumed meal, erroneous estimates occur frequently, resulting in incorrect dose calculations.

If the dose delivered is smaller than the needed dose, an insufficient amount of medication is delivered to the person. In the case that the medication is insulin, this may result in the person suffering hyperglycaemia, thereby increasing the risk of long term complications. On the other hand, if the dose delivered is larger than the needed dose, an excessive amount of medication is delivered to the person. In the case that the medication is insulin, this may result in the person suffering hypoglycaemia, the person thereby being at risk of suffering the known consequences thereof.

Furthermore, in the case of insulin administration, the estimated nutritional composition of a meal - in particular the carbohydrate content of the meal - is a rather decisive factor for the correct calculation of the correct dose of insulin. Any uncertainty in the estimate of the carbohydrate content may result in a less precise determination of the estimated required dose of insulin. Unfortunately few people are able to correctly estimate their meals. This difficulty has previously been acknowledged and several attempts have been made to improve the dose calculation.

Graff, Gross, Juth, and Charlson have examined how well people on intensive insulin therapy are at counting carbohydrates[1]. They found that on average, the carbohydrates contained in the breakfast were overestimated by 8.5% (-93% to +100%) and underestimated for lunch by -28% (-97% to +43%), for dinner by -23% (-95% to +80%) and for snacks by -5% (-96% to +122%). When the respondents' estimated meal boluses were compared to the correct meal boluses (calculated using each respondent's reported target blood glucose, insulin sensitivity and insulin to carbohydrate ratio), subjects, on average, overestimated their insulin needs for a pre-meal blood glucose of 60 mg/dL by 0.8 ±3.74 IU for a pre-meal blood glucose at their target by 0.7 ±4.7 IU. Graff et al. concluded that these results suggest that estimates of carbohydrate content of meals are quite inaccurate, even among individuals who regularly use carbohydrate counting. The study covered 64 people, 35 of which had type 1 and 29 had type 2 diabetes. Other studies have shown that obese people with type 1 report the meal content to be 66% of the actual content while normal weight people report 90% [2].

US2004180810A1 discloses a method of food and insulin dose management. The patient figures out how many toasts there are going to be in a meal and takes insulin to match. After the meal, the patient measures BG-values so as to verify that the system is working as planned. If the blood sugar does not react as expected, the estimation of carbohydrates may be inaccurate.

US2005065760 discloses a method for guiding a user to select a dose of insulin. An adaptive correction factor takes into account the user's proficiency in estimating carbohydrates contained in a meal.

US2003114836 discloses a bolus estimator that estimates a bolus based on carbohydrate consumption after the user has demonstrated a sufficient understanding of how to estimate carbohydrate intake.

US 2006272652 describes a virtual patient software system for educating individuals with diabetes. The selected patient model takes into consideration that a user often underestimates and overestimates the number of grams of carbohydrates that a patient consumes.

WO0010628 is concerned with an external infusion device and the user can utilize a bolus estimator information to learn' insulin sensitivity values, carbohydrate counting, etc.

WO0018293 describes a diabetes management system which predicts a future blood glucose value of patient and recommends corrective action when predicted value lies outside target range.

Hence, even though the difficulty of estimating the correct carbohydrate content of a meal and its influence on the correct insulin dosage has been acknowledged, there remains a need for a method and apparatus that assist a user in improving the accuracy of the estimates of a nutritional parameter, e.g. the carbohydrate content, of a meal in connection with administration of a medication.

SUMMARY

According to one aspect, an apparatus for monitoring administration of a medication to a user comprises processing means adapted to:

- obtain input values indicative of at least a first and a second measurement of a physiological parameter of the user, and of at least a dose of medication administered to the user;
- determine, from the obtained input values, an estimate of a nutritional parameter of a meal consumed by the user between the first and the second measurement;
- generate an output indicative of the determined estimate.

Hence, embodiments of the apparatus described herein improve the user's ability to correctly estimate relevant nutritional parameters of meals, such as the carbohydrate content of the meal. In the context of diabetes, incorrect estimated meals are a severe problem and a major contribution to incorrect insulin dosage, as most people underestimate meals and since the carbohydrate content of a consumed meal has great impact on the calculation of insulin dosage. Furthermore, the correction of meal estimates as described herein can be used with physiological models to obtain higher accuracy of meal estimates and bolus insulin calculation.

These factors, i.e. the fact that the meal estimate mainly depends on the user, and the fact that it contributes strongly to the amount of insulin calculated, cause an improved support for the user in the meal estimate to be very advantageous.

Furthermore, the method and apparatus described herein facilitate calculating the dose or series of doses of medication in a user-specific, personal manner.

Furthermore, the method and apparatus described herein facilitate adapting the calculation of the dose or series of doses of medication to changes in the behaviour of the person to receive the dose or series of doses of medication.

The dose or series of doses of medication may be liquid dose(s) of medication, for example for subcutaneous or intravenous delivery, e.g. by means of an injection device or an infusion device. For example, the medication may be administered by means of an injection device, such as an injection pen or a needle-less injection device, a nasal spray, orally, e.g. in tablet form or in liquid form, or in any other suitable way. The apparatus described herein may thus form part of or be connected to an injection device, an infusion device, such as a patch infusion pump, or another suitable drug delivery device.

The input values may be obtained in various ways, depending on the nature of the respective individual parameters. Thus, the input values may be supplied directly to an input device of the apparatus from another device adapted to measure and/or store relevant parameter values, or the apparatus may comprise a measurement device, in which case the relevant input values may be obtained directly by measurement. Alternatively or additionally, some input values may be entered manually by a user. Additional input values may be automatically generated by the apparatus and/or originally set, i.e. they may be entered initially and normally never changed or at least only changed very rarely.

Thus, the input values may be obtained by measurement, by manually entering values, by data transfer, e.g. using a hardwired or wireless communications interface, such as by means of an infrared or a radiofrequency link, by calculation, by retrieving data from internal storage means, etc.

The input values of the first and second measurement of the physiological parameter are typically measured prior and after intake of a meal. They may be measured immediately before and after a meal intake, respectively, or at a point in time within respective periods of time prior and after meal intake. The physiological parameter may also be measured repeatedly or even continuously over a period of time prior and/or after meal intake. Furthermore, one of the measurements may at least partially be performed during meal intake.

Similarly, the input value of at least a dose of medication administered to the user may be obtained by the apparatus directly via an input interface of the apparatus, entered manually by the user via a suitable user interface of the device, or in any other suitable way.

For example, the apparatus may include a user-input device such as a dial, a push button, or any other suitable device for receiving a user input, so as to allow a user to manually input information about an administered insulin bolus and/or the like. Alternatively or additionally, the apparatus may obtain the administered dose directly from a drug delivery device having a suitable output interface for communicating the administered dose of medication. The dose of medication may be administered in a predetermined period of time around the meal intake, e.g. prior to the meal intake.

In case of diabetes, the measured physiological parameter may be the blood glucose level of the user which may be measured in any suitable way known in the art. Similarly, in the case of diabetes, the medication may be insulin and/or another medicament for treatment of diabetes. In this case the medication may for instance be inhalable insulin or injectable insulin. Alternatively it may be other kinds of medication for regulating blood glucose or insulin sensitivity, e.g. GLP-1 like hormones or oral drugs.

Alternatively, the dose or series of doses may be for anticoagulation treatment. In this case the medication may be warfarin or phenprocoumon. Phenprocoumon is a vitamin K antagonist that inhibits coagulation by blocking synthesis of coagulation factors. During anticoagulation treatment the patient regulated medication by taking a blood sample that is analysed in an apparatus. From this the next dosing can be calculated. Too large a dose can cause too little coagulation, thereby causing bleedings that can be dangerous. A too small dose can cause thrombosis. The needed dose depends on the amount of K-vitamin in the patient's food. Products from the cabbage family contribute a lot.

Generally, representative medications include pharmaceuticals such as peptides, proteins, and hormones, biologically derived or active agents, hormonal and gene based agents, nutritional formulas and other substances in both solid (e.g. dispensed) or liquid form. In the description of the exemplary embodiments reference will be made to the use of insulin.

Typically, a user may perform a measurement of the blood glucose level prior to the meal intake, determine a suitable bolus dose of insulin based on the measured blood glucose level and on an - explicit or implicit - estimate of the carbohydrate content of the meal to be consumed, and administer the determined dose prior to the meal intake.

The determination of an estimated value of a nutritional parameter of the meal is performed based on at least the obtained input values, i.e. from at the least the measured physiological parameters prior and after meal intake and from the administered dose of medication. Consequently, the user is provided with feedback about the actual nutritional content of the consumed meal. This in turn facilitates an improvement of the user's own initial estimates or guesses of the nutritional content of the meal that are the basis for the calculation of the administered dose of medication. Consequently, the apparatus described herein facilitates a long-term improvement of the appropriate medication of the user. The carbohydrate content of the meal is an example of a nutritional parameter. The carbohydrate content is a particularly important parameter in the context of calculating doses of medication in the context of diabetes.

In some embodiments, the estimate of the nutritional content is determined from a physiological model of the user, e.g. a physiological model for modelling the physiological processes involving the influence of the nutritional content of the meal and of the medication on the measured physiological parameter. When the physiological model is a user-specific model, a more accurate estimate for the particular user may be determined. For example, the physiological model may include one or more model parameters that may be determined for each user and stored in the apparatus. The physiological model may be a physiological prediction model for predicting a value of the physiological parameter after the meal intake from at least the value of the physiological parameter before the meal intake, from the dose of medication administered to the user, and from the estimated nutritional parameter of the meal. The estimate of the nutritional parameter may be determined from such a predictive model by iteratively calculating the predicted value of the physiological parameter after the meal intake for different preliminary estimates of the nutritional parameter of the meal, and by determining the estimated nutritional parameter as a value of the nutritional parameter that results in a best correspondence of the corresponding predicted value of the physiological parameter after the meal intake with the actually measured value of the physiological parameter after the meal intake.

When the estimate of the nutritional parameter is determined as a value of the nutritional parameter for which a difference between the predicted value of the physiological parameter and the measured value of the physiological parameter after the meal intake is smaller than a threshold, a particularly accurate estimate is provided. For example, such an estimate may be determined my means of any suitable numeric minimisation algorithm for minimising the difference between the predicted and measured values with respect to the estimate nutritional parameter.

The apparatus may further comprise an output device for generating a user-detectable output indicative of the determined estimate. For example the apparatus may include a display for displaying the determined estimate or an output value derived from the determined estimate to the user, or an output device for providing any other user detectable output, e.g. a print-out, an audible output or the like. The output may indicate the estimated nutritional parameter in any suitable way, e.g. as a an absolute or relative content, as a level of adequacy compared to a previously entered estimate, e.g. as a binary signal corresponding to "adequate" and "inadequate," or as a multilevel degree of adequacy having more than two possible values or even according to a continuous scale.

Alternatively or additionally, the apparatus may include a data communication interface for communicating the determined estimate or a value derived thereof to another device or system, e.g. to a data processing system such as a PC. Examples of data communications interfaces include wired or wireless interfaces, e.g. a serial interface, an USB interface, a short-range radio-frequency interface such as Bluetooth, an infrared interface, or the like. Further examples include removable storage media such as memory cards, memory sticks, diskettes, etc. Consequently, estimated values for a plurality of meals may be communicated to another device or system for further analysis, e.g. by health care personal.

It will be appreciated that the apparatus may further be adapted to obtain additional input values, such as the type of medication, the type of meal (e.g. "lunch," "dinner," "snack", etc.), the time of measurement of the physiological parameter, the time of administration of the medication, the time of the meal intake, and/or the like. Some values may serve as additional input to the estimation of the nutritional parameter, e.g. as input to a physiological model. Alternatively or additionally, some of these additional input values may be stored by the apparatus in relation to the estimated value of the nutritional parameter, and/or communicated to another device for further analysis.

In some embodiments, the processing means is further adapted to receive an input value indicative of a preliminary estimate of the nutritional parameter or a value derived from the preliminary estimate of the nutritional parameter. The apparatus may thus generate an output indicative of the deviation of the preliminary estimate from the estimate subsequently determined by the apparatus based also on the measured physiological parameter after meal intake. Consequently, the user is provided with an indication of the accuracy of the initial estimate, thus facilitating a gradual improvement of the estimates performed by the user.

The apparatus may output a deviation for each estimate for which a preliminary estimate is entered. Alternatively, the apparatus may output a deviation only if the deviation exceeds a predetermined margin. Alternatively or additionally, the apparatus may determine an average or a trend of the deviation for a plurality of meals, or for a plurality of meals of a certain type, and output the determined average or trend, thus reducing the effects of random deviations, inaccuracies of the determination of the estimate, or other error sources that may be attributed to "noise."

Meals of a certain type may be meals that are indicated as a certain type or class of meal by the user, e.g. as being a "breakfast", "lunch" etc. Alternatively or additionally, the meals of a certain type may be meals consumed at a predetermined time of day, meals having a preliminary estimate of the nutritional parameter within a predetermined interval, and/or the like. Consequently, the user may be provided with valuable feedback indicating the user's ability to correctly asses/estimate certain types of meals, e.g. lunches, meals with high carbohydrate content, etc.

Alternatively or additionally, the entered preliminary estimate may be used as a starting value for an iterative calculation of the estimated value of the nutritional parameter.

When the processing means is further adapted to calculate a dose or a series of doses of medication based on at least the obtained input value of the physiological parameter of the user measured prior to intake of the meal by the user, an apparatus is provided that may be used both as a dose/bolus calculator and as a monitoring device that provides feedback to the user about the appropriateness of the administered doses. In some embodiments the does calculation is further based on an input value indicative of a preliminary estimate of the nutritional parameter.

The dose or series of doses of medication may be calculated based on one or more further input parameters. Accordingly, the dose or series of doses of medication may be calculated on the basis of an image of the person to receive the medication which is as close to the reality as possible. Thereby the calculated dose or doses will be more likely match the need for medication for that specific person at that specific time, i.e. a highly customized calculation is provided. This is very advantageous, because the adverse effects associated with incorrect doses are thereby minimised.

Furthermore, a comparison between different input values, e.g. different input values received in various manners such as manually, originally set, automatically generated, etc., may reveal alterations in normal behavioural pattern, and thereby such alterations may be taken into account when the dose or doses is/are calculated.

The apparatus may be adapted to calculate a bolus dose of medication, i.e. the dose or series of doses of medication may be a bolus dose. In the present context the term 'bolus dose' should be interpreted to mean a single dose of drug which is to be delivered over a short period of time. It may, e.g., be an extra amount of insulin taken to compensate for an expected rise in blood glucose level, e.g. in connection with consumption of a meal.

Embodiments of the present invention can be implemented in different ways, including the apparatus described above and in the following, further methods, systems, devices and product means, each yielding one or more of the benefits and advantages described in connection with the first-mentioned apparatus, and each having one or more embodiments corresponding to the embodiments described in connection with the first-mentioned apparatus and/or as disclosed in the dependent claims.

In particular, disclosed herein is a method of monitoring administration of a medication to a user, the method comprising:

- obtaining input values indicative of at least a first and a second measurement of a physiological parameter of the user, and of at least a dose of medication administered to the user;
- determining, from at least the obtained input values, an estimate of a nutritional parameter of a meal consumed by the user between the first and the second measurement;
- generating an output indicative of the determined estimate.

It is noted that the features of the method described above and in the following may be implemented at least in part in software and carried out on a data processing device or other processing means caused by the execution of program code means such as computer-executable instructions. Here and in the following, the term processing means comprises any circuit and/or device suitably adapted to perform the above functions. In particular, the above term comprises general- or special-purpose programmable microprocessors, Digital Signal Processors (DSP), Application Specific Integrated Circuits (ASIC), Programmable Logic Arrays (PLA), Field Programmable Gate Arrays (FPGA), special purpose electronic circuits, etc., or a combination thereof.

Hence, according to one aspect, a computer program product comprises computer program means adapted to cause, when executed on a data processing system, the data processing system to perform the steps of the method described herein.

BRIEF DESCRIPTION OF THE DRAWINGS

The above and other aspects will be apparent and elucidated from the embodiments described in the following with reference to the drawing in which:

Fig. 1 shows a block diagram of an example of an apparatus for monitoring administration of a medication.

Fig. 2 shows an overall flow diagram of an example of a process for monitoring administration of a medication.

Fig. 3 shows an overall flow diagram of another example of a process for monitoring administration of a medication.

Fig. 4 illustrates a physiological model of glucose homeostatsis for a person with type 1 diabetes.

Fig. 5 illustrates examples of prediction results obtained from a physiological model.

Throughout the drawings, equal reference signs refer to equal or corresponding elements, features, or components.

DETAILED DESCRIPTION

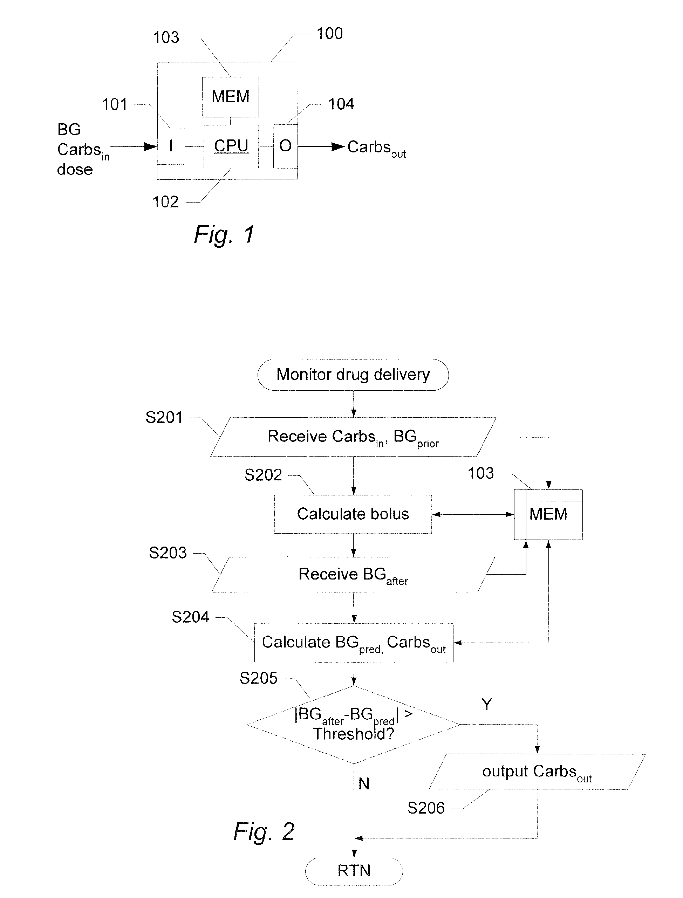

Fig. 1 shows a block diagram of an example of an apparatus for monitoring administration of a medication. The apparatus, generally designated 100, comprises one or more input devices 101 for receiving input values, a processing unit 102, a memory 103, and one or more output devices 104.

The input device(s) 101 includes a user-interface allowing a user of the apparatus to enter data, such as a estimated carbohydrate content ('Carbs$_{in}$') of a meal, measured blood glucose levels (BG), administered bolus doses ('dose'), etc. The user interface may include any suitable device for entering data, such as push-buttons, dials, a keypad, a touch screen, and/or the like. Optionally, the input device(s) 101 may further include one or more data interfaces for directly receiving data from other devices, such as blood glucose measuring devices, drug delivery devices, data processing systems, and/or the like. The data interface. Examples of such data interfaces include wired and wireless interfaces, e.g. serial interfaces, USB interfaces, infrared interfaces, short-range radio-frequency interfaces such as Bluetooth, local area network interfaces, cellular telecommunication interfaces, and/or the like. Other examples of data interfaces include removable data carriers such as memory sticks, memory cards, optical discs, tapes, magnetic discs, and/or the like.

Input values supplied directly to the input device 101 of the apparatus or obtained from a measurement device forming part of the apparatus may include a measured blood glucose (BG). The apparatus may be adapted to cooperate with means for measuring a glucose level for the person to receive the dose or the series of doses of medication, and the result of a glucose measurement may be obtained directly by the apparatus via the input device. The glucose level may, e.g., be blood glucose (BG) level which is normally measured by means of a BG measurement device. Alternatively, the glucose level may be a plasma glucose level which may be measured substantially continuously from blood plasma, e.g. by means of Continuous Glucose Monitoring (CGM). Similarly, a delivered or calculated dose of insulin may be received from an insulin delivery device such as an insulin pen or an insulin pump, or from a bolus calculator, respectively. Further examples of such input values include, but are not limited to a current activity level, development in one or more measured parameters over a specified previous time interval, etc. For example, the apparatus 100 may further comprise or receive data from an accelerometer attached to or worn by the person to receive the dose or series of doses of medication. The accelerometer being adapted to provide information relating to the activity level of said person to the apparatus via the input device. Alternatively, the input device may be adapted to communicate with an accelerometer which does not form part of the apparatus.

In the case that development in one or more measured parameters is supplied to the input device, such information is preferably retrieved from internal or external storage means, e.g. from an external computer in which information relating to previous measurements, manually entered values or previously calculated values are stored.

Values which are entered manually may, e.g., be values measured by a separate device or values which are estimated by the user. Measured values may include any of the values defined above, the only difference in this case being that instead of supplying the values directly to the input device, the user enters the values manually. Estimated values may, e.g., relate to the contents and/or amount of an upcoming meal, e.g. an estimated carbohydrate content ('Carbs$_{in}$'), to an upcoming intake of alcohol, or to an activity level, e.g. in case the person is about to considerably increase the activity level for a period of time, e.g. by performing some kind of exercise.

Values which are automatically generated are preferably of a kind which the device is capable of obtaining based solely on information which is readily available to the apparatus, i.e. without the requirement for additional information being supplied to the apparatus. For instance, the apparatus may be provided with a clock or another kind of time measurer, and the apparatus will thereby 'know' the time of the day, week month, etc. Accordingly, 'time' is an example of a value that may be automatically generated. Based on this information, possibly combined with information relating to a normal behavioural pattern of the person, the apparatus may be capable of generating various input parameter values, such as expected food intake, expected activity level, age of a catheter in case the apparatus is an infusion device, age of the person, etc. In the case that the apparatus comprises or is connected to a measurement device, a measured value may also be regarded as an automatically generated value. Furthermore, the time of receipt of blood glucose measurement data, insulin dose data, and/or carbohydrate estimates may be directly recorded by the apparatus and used in the physiological prediction model as described herein.

Values which are originally set may be values which are specific for the person to receive the dose or the series of doses of medication, but which are normally not subject to variations as a function of time, or which vary only slowly as a function of time, including normal behavioural patterns for the person. Examples of such values include, but are not limited to, race, sex, initial age, medical condition, e.g. kind of diabetes, target glucose level, e.g. target BG level (BG$_{target}$), one or more Carbohydrate-to-Insulin Ratios (CIR), one or more Insulin Sensitivity Factors (ISF), normal food intake as a function of time, normal activity level as a function of time, body weight, body mass index, etc. It is clear that some of these values do not change. This is, e.g. the case for race and sex. A value 'initial age' may define the age of the person to receive the dose or series of doses of medication at the time where the apparatus is initially set. The apparatus may be capable of automatically generating the current age of the person based on the initially entered age in combination with information relating to time/date. However, some of the values may vary over time, albeit only very slowly. This is, e.g., the case for body weight, body mass index, normal food intake and normal activity level. It should, therefore, be possible to alter these values when changes happen, in order to ensure that the values available to the apparatus, at any time, are as close to reality as possible.

A 'normal behavioural pattern' for a person may include information relating to activity level and/or food intake for the person, including periodical variations as a function of time, e.g. food intake at various habitual meals during a normal day, variations in meal times between working days and weekends, varying activity levels during the course of a day and/or during a week, etc.

For example a 'normal food intake pattern' may include information relating to the expected food intake as a function of time, e.g. the time of day, the time of week, etc. The 'normal food intake pattern' may specify a number of customary meals during a day, the normal times or time intervals at/during which each customary meal is usually consumed, and/or one or more properties such as a parameter (or interval) indicative of the nutritional composition, e.g. the carbohydrate content, of each meal.

Similarly, a 'normal activity level' may include information relating to the expected activity level as a function of time, e.g. the time of day, the time of week, etc. Thus, the 'normal activity level' may specify that the person normally rides a bicycle to and from work Monday to Friday, and that the person attends a workout class every Tuesday from 4 pm to 6 pm, etc.

The apparatus may be adapted to compare automatically generated input parameter values, received input parameter values and originally set input parameter values. Thus, the apparatus is capable of comparing information obtained in various manners, and thereby an image of the conditions for the person to receive the medication, which is as close the reality as possible, can be obtained.

The processing unit 102 may include a suitably programmed general- or special-purpose programmable microprocessor, a Digital Signal Processors (DSP), an Application Specific Integrated Circuit (ASIC), special purpose electronic circuits, etc., or a combination thereof. In particular, the processing unit is suitably programmed, e.g. by means of program code stored in memory and loaded from the memory 103, to perform the monitoring process described in connection with figs. 2-5 below.

The memory 103 may include any suitable storage medium for storing program code and data, such as model parameters of a physiological model, received input data, and/or the estimated nutritional parameters and/or other outputs generated by the device. Examples of suitable storage media include a Random Access Memory (RAM), a Read Only Memory (ROM), a flash memory, an EPROM, an EEPROM, and/or the like. The memory 103 may further include more than one memory device, e.g. a RAM, a ROM and a flash memory.

The output device(s) 104 includes a display for displaying the output calculated by the apparatus 100, in particular an estimated carbohydrate content of a previously consumed meal ('Carbs$_{out}$'), or any other suitable output means. Alternatively or additionally, the output device (s) 104 may include one or more data interfaces for directly transmitting data to other devices or systems, such as to a data processing system of a health care centre, to a conventional computer, and/or the like. Examples of suitable data interfaces have been described in connection with the input device 101. It will be appreciated that the apparatus 100 may use the same or different data interfaces as input and output devices, respectively. Other means for providing a user-detectable output signal may comprise a display, an LED, or other visible indicator, vibratory elements or electro-muscle stimulation (EMS).

For example, the apparatus 100 may be embodied as a hand-held or user-worn device. For example, the apparatus 100 may be embodied as a suitably programmed general purpose electronic processing device such as a mobile terminal, a personal digital assistant (PDA), a suitably programmed hand-held computer. Alternatively, the apparatus 100 may be a special-purpose medication monitoring device, e.g. having one or more data communication interfaces for receiving data directly from other devices, such as a blood glucose measuring device, a drug delivery device, and/or the like. In some embodiments, the apparatus may be an integrated device which also includes a drug delivery device and/or a blood glucose measuring device and/or a bolus calculator and/or the like.

Operation of a monitoring device as described herein, e.g. the device shown in fig. 1, will now be described with reference to figs. 2-5, and with continued reference to fig. 1.

Fig. 2 shows a flow diagram of an example of a process for monitoring insulin administration. Embodiments of the process may be performed by the processing unit 102 of the apparatus 100.

In initial step S201, the apparatus 100 receives via input interface 101 a blood glucose measurement (BG$_{prior}$) indicative of the blood glucose level of the user prior to a meal intake. When the glucose level has been measured, the result may be communicated to the input device via a data communication channel, or it may be entered manually. Alternatively, the measurement apparatus may form part of the apparatus 100. The apparatus 100 further receives via input interface 101 input values indicative of an estimate of the carbohydrate content (Carbs$_{in}$) of the meal to be consumed. The process stores the received values in memory 103.

In step S202, the process calculates a bolus insulin dose to be administered to the user in connection with the meal intake. The calculation may be based on the following parameters retrieved from memory 103:

'Carbs' is a parameter relating to food intake. 'Carbs' may be expressed as the amount of total grams of carbohydrate in the meal, i.e. the received estimate of the carbohydrate content of the meal. 'Carbs' is preferably an estimate of the amount of carbohydrates contained in a meal which the person to receive the dose is about to consume. The user himself or herself may perform the estimate manually. However, the apparatus may be capable of calculating a preliminary estimate from information delivered by the user concerning the meal. Such information may include types of food, such as 'vegetables', 'fish', 'pasta', 'meat', etc., weight of each food type, etc.

'CIR' is a parameter relating to an amount of medication corresponding to a specific food intake. In particular, 'CIR' (Carbohydrate-to-Insulin Ratio) is a personal relation reflecting the amount of drug needed in response to a specific amount of food. In case the medication is insulin for treating diabetes, the CIR parameter may preferably be a measure for how many grams of carbohydrate corresponds to one unit of fast acting insulin. This parameter is preferably stored in memory 103. However, the CIR parameter may depend on the time of the day, and it may therefore be stored in the form of a periodical function of time. For many people the value of CIR depends on the time of the day, and for women it may also depend on the time of the month, relatively to the menstruation cycle. At initialisation of the apparatus the known values of the parameter for the person are entered and the apparatus may, based on that among other things, suggest values to use at any given time. This could for instance be obtained by forming/interpolating a smooth curve through the known values. For instance, one person may know that breakfast CIR is 5 g/IU insulin and that CIR for the remaining part of the day is 8 g/IU insulin. In this context 'breakfast' should be understood as the first meal of the day if it is consumed before noon. Many women are less sensitive to insulin immediately prior to menstruation. Accordingly, for one person it may be known that three days before expected menstruation the CIR values are approximately 20% less than normally, i.e. in the example given above the breakfast CIR is 4 g/IU insulin, and the CIR for the remaining part of the day is 6.4 g/IU insulin. If only one value for CIR is initially known, the apparatus may help suggesting values for other periods of the day. For instance, the CIR value for one person may have been measured to be 10 g/IU insulin at 2 pm. The apparatus may then suggest using 8 g/IU insulin for breakfast CIR and 10 g/IU insulin for CIR at any other time of the day.

$BG_{current}$ and $BG_{target}$ are parameters reflecting the current blood glucose level and a target blood glucose level, respectively. $BG_{current}$ reflects the current BG level for the person to receive the dose of medication, and it is preferably a measured value of the BG level. $BG_{current}$ is preferably supplied to the apparatus as described above. $BG_{target}$ reflects a desired target value for the BG level for the person to receive the dose of medication. Thus, $BG_{target}$ may be a constant value for a given person, in which case it may advantageously be initially set once it has been determined for the relevant person and stored in memory 103. Alternatively, $BG_{target}$ is a periodical function of time, e.g. reflecting variations in the target value for BG during the day, and/or on a longer time scale, e.g. reflecting variations arising from the menstruation cycle for a woman. For instance, $BG_{target}$ could be 4-8 mM during daytime, 6-8 mM at bedtime and 6-10 mM immediately prior to performing some kind of exercise. Furthermore, in case the person is pregnant a somewhat lower target value may be chosen, such as 4-5 mM prior to consuming a meal. Accordingly, the term $BG_{current}$-$BG_{target}$ is the difference between the current BG level and the desired target BG level, and it therefore reflects a possible needed correction.

'ISF' is a parameter relating to medication sensitivity. In particular, 'ISF' (Insulin Sensitivity Factor) is a personal relation describing the impact on the BG level of a specific dose of medication. In case the medication is insulin for treating diabetes the ISF parameter may describe how large a drop in BG level one unit of insulin gives raise to (e.g. as measured in mM/IU.) The parameter ISF may be stored in memory 103.

'IOB' (Insulin On Board) is a parameter relating to an amount of medication still present from a previously delivered dose. However, only the part of the remaining amount of medication which is not related to a meal should be counted in. Thus, medication still on board which counteracts a meal which is also still on board should not be counted in, i.e. it is 'cancelled out' by the meal. The IOB parameter is preferably a function of several factors, such as medication dose size, medication type (e.g. insulin type, such as fast acting or slow acting), age, body mass index, type of disease or condition (e.g. type I or type II diabetes), race, time of day, age of catheter (in case of infusion apparatus), anatomic site of catheter insertion, time in menstruation period, amount of exercise in the near past or near future, training state, change of time zone, stress, alcohol intake, etc. All of these factors have an impact on how fast the medication is consumed by the body. Thus, the IOB may advantageously be calculated in response to a number of input parameter values, and/or in response to a comparison of two or more input parameters as described herein.

Thus, at least the CIR, ISF and IOB parameters may be regarded as personal parameters. In the present context the term 'personal parameter' should be interpreted to mean a parameter which reflects one or more specific characteristics for a specific person, such as age, sex, race, sensitivity to drugs, food absorption, etc. At least the CIR, ISF and IOB parameters may also be regarded as adaptive parameters. In the present context the term 'adaptive parameter' should be interpreted to mean a parameter which can be modified or adjusted by the apparatus in response to input parameter values supplied to the apparatus. An example of an adaptive parameter is 'activity level' as described above. In the example given above the expected activity level (attending a workout class) is modified in response to input data from an accelerometer.

It will be appreciated that the above parameters may alternatively be input and/or used in different units. For example, the carbohydrate content may alternatively be measured in "BE" ("Broteinheiten"), "KE" ("Kohlenhydrateinheiten"), or the like.

Based on the above parameters, the dose is calculated on the basis of a term reflecting the relationship between food intake and food absorption, a term reflecting the impact of delivered medication relatively to a desired objective, and a term reflecting the amount of medication which is still present from a previously delivered dose. In particular, the process may calculate the bolus insulin from the following equation:

$$Insulin = \frac{Carbs}{CIR} + \frac{BG_{current} - BG_{target}}{ISF} - IOB$$

(E0)

wherein 'Insulin' is the calculated dose, and the remaining parameters are the parameters described above. The above equation is thus calculated based on parameters received by the apparatus in step S201 and/or based on stored parameters retrieved from memory 103, e.g. parameters such as age, sex, CIR, and/or the like.

It will be appreciated that other methods for calculating an insulin dose may be used. For example, in one alternative method, the parameter 'carbs' may further be multiplied by a 'Food Speed Index' (FSI) which reflects how fast the person to receive the dose or series of doses of medication absorbs the specific food. It preferably also reflects the resulting effect on the blood glucose level. This parameter depends on the composition of the meal, in particular the amount of fluid, fat and fibre. It may preferably be entered by the user, e.g. in the form of a selection between 'slow', 'medium' or 'fast', or as an estimated number. Examples of slowly absorbed food are types of food having a relatively large fat content, or complex carbohydrates. Examples of fast absorbed food are candy or cornflakes. For slowly absorbed food the FSI parameter will be smaller than 1, and for fast absorbed food the FSI parameter will be larger than 1, and the FSI parameter preferably ranges between 0.6 and 1.4. Accordingly, the FSI parameter reduces or increases the 'Carbs' parameter in order to take the kind of carbohydrates consumed as well as the expected impact on the BG level into account. In the case that the FSI parameter can not be estimated, it may be set to 1, thereby assuming that the carbohydrate content indicated by the 'Carbs' parameter is of an average kind in terms of absorption, i.e. 'medium' rather than 'slow' or 'fast'.

At least one of the input parameters may relate to activity level for the person to receive the dose or series of doses of medication. Such input parameters may include, but are not limited to, an originally set schedule for normal activity level, actual activity level measured by means of an accelerometer, and manually entered estimated activity level. It is an advantage that the activity level for the person is taken into account, in particular in the case that the medication is for treating diabetes, because activity level has a great impact on the amount of required medication.

Some people may often skip the calculation and rather base their insulin dosing on assumptions as "similar to yesterday". A bolus calculator has the mentioned factors as input and then performs the calculation based on the preset conversion factors. Current blood glucose can be transferred automatically from the blood glucose meter and target blood glucose, CIR and ISF are stored in the memory, so the only thing that has to be entered is the estimate of carbohydrate contents in the meal.

In subsequent step S203, the process receives a blood glucose measurement (BG$_{after}$) indicative of the blood glucose level of the user after the intake of the meal. The blood glucose measurement may be performed at a suitable time after meal intake where the influence of the consumed meal and the insulin administered in connection with the meal on the blood glucose level are measurable. For example, the blood glucose measurement may be performed at a predetermined period of time after meal intake, e.g. between ½ hour and 2 hours after meal intake.

In subsequent step S204, the process calculates an expexted/predicted blood glucose value (BG$_{pred}$) at a predetermined time after meal intake based on the blood glucose measurement prior to the meal intake, the administered dose of insulin and the estimate of the carbohydrate content of the consumed meal, and using a suitable physiological model. In particular, the process may calculate the expected/predicted blood glucose level for the time at which the actual post-meal measurement of the blood glucose level was performed. For example, the time of measurement may be entered (or received from another device) together with the measured blood glucose level. Alternatively, the apparatus may record the time of entry/receipt of the measured value, and use this time as an approximation of the time of measurement.

A physiological prediction model can be constructed in various ways known as such in the art, and several models have been reported in literature. Research has shown that the meal estimate statistically is the major source for discrepancies between the expected and measured post-meal blood glucose levels (see e.g. Kildegaard J, Randløv J, Poulsen JU, Hejlesen OK. : The impact of non-model related variability on blood glucose prediction, Diabetes Technology and Therapeutics, 2007.) Hence, if the weekly carbohydrate intake deviates, in particular, if this is this case over a certain period of time, e.g. a week, an incorrect carbohydrate estimate is a likely cause for this deviation.

An example of a physiological model may be constructed by combining an insulin model by Berger [3], a meal model by Lehmann [4] and the minimal model by Bergman [5]. Hence, useful meal estimation feedback is possible based on a relative simple model. A more comprehensive model may give more accurate results, in particular outside the normal BG range. For example, the minimal model has been accepted as being a gross simplification outside normal BG range [6].

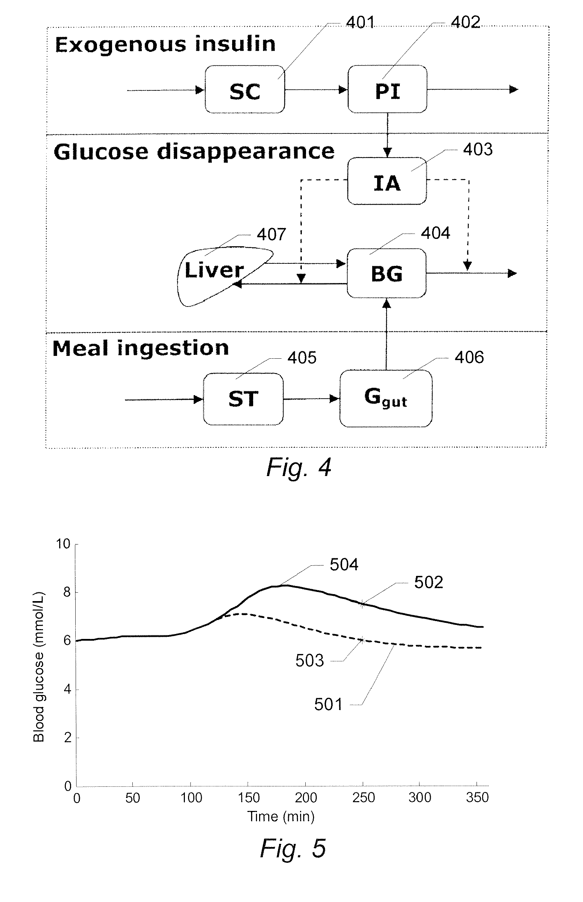

Fig. 4 shows an example of physiological compartment model of glucose homeostasis for a person with type 1 diabetes. The model includes six compartments 401-506. Four of the compartments may be described directly by differential equations. These are plasma insulin PI (402), insulin action IA (403), blood glucose BG (404) and gut glucose content $G_{gut}$ (406). Subcutaneous insulin level SC (401) and the stomach contents of glucose ST (405) can be derived, but are not, however, of main interest in the present context. They are used mathematically to store the current content which is updated if more insulin is injected or carbohydrates are eaten. The liver (407) is modelled in equation (E3) below, where it is part of both the positive and negative contributions.

The model receives information as inputs about carbohydrate contents of one or more meals, the amount, type and time for insulin injection or infusions and blood glucose values at one or more times. The user's physiological values (blood glucose, gut contents, plasma insulin) are then simulated by iterating a number of differential equations modelling the respective compartments over one or more time steps forward in time until new values are entered or until a predetermined time corresponding to the time at which the measured blood glucose value after meal intake was obtained.

Still referring to fig. 2, the process may e.g. iteratively calculate the predicated blood glucose level for different input estimates of the carbohydrate content, so as to determine a best fitting estimate of the carbohydrate content of the meal ($Carbs_{out}$) by simulating a number of different meal estimates. For example, the process may perform a minimisation process, so as to minimize the difference between the predicted and the measured blood glucose value. In some embodiments, the process may initially calculate the predicted blood glucose value based on the originally received estimate, and perform the iterative calculation only if the deviation to the measured value is larger than a predetermined threshold.

It will be understood that the apparatus may have stored therein a look-up table of pre-computed predicted values for a range of input parameters. The calculation of the predicted blood glucose value may thus be based in total or in part of the pre-computed values, e.g. by interpolating between values in the look-up table.

Alternatively other non-physiological based methods, e.g. a neural network trained on the BG difference, the insulin taken, the CIR and the ISF can be used for the calculation of the predicted blood glucose.

Alternatively other physiological models, e.g. a rule-based model may be used based on the BG difference, the insulin taken, the CIR and the ISF. For example, an algorithm for calculating an insulin bolus as described above may be inverted and used with the meal estimate as the unknown. In particular, equation (E0) above may be used to calculate the carbohydrate content of a meal that results in a target BG value ($BG_{target}$) equal to the actual measured value after the meal intake. Such a rule-based model may also be implemented using a lookup table. However, the advantage of a more elaborate predictive physiological model is that other factors may be included (like exercise), and it will work with CGM data as well.

In step S205, the calculated expected blood glucose value ($BG_{pred}$) based on the original estimate of the carbohydrate content ($Carbs_{in}$) is compared to the measured blood glucose value ($BG_{after}$) after meal intake. If there is a large discrepancy, e,g, larger than a predetermined threshold, the process continues at step S206 and generates an output indicating the discrepancy and the actual estimated value to the user, e.g. by displaying the user's original estimate ($Carbs_{in}$) and the estimate ($Carbs_{out}$) calculated in step S204 to the user via output interface 104. Alternatively or additionally, the process may store the original estimate ($Carbs_{in}$) and the calculated estimate ($Carbs_{out}$), optionally with further information related to the meal, such as the time of the meal, the type of the meal, the input parameters that were the basis for the estimation, and/or the like. In this case the process may calculate a moving average of discrepancies over a plurality of meals or a plurality of meals of the same type. If the average discrepancy is larger than a predetermined threshold, the process may generate an output indicating to the user that on average the user tends to under- or overestimate the carbohydrate content of the meal by the calculated average discrepancy. It will be appreciated that alternatively or additionally, the process may perform other analyses steps on the calculated estimates, e.g. for detecting trends and/or patterns in the users accuracy when estimating the carbohydrate content. Alternatively or additionally, the process may communicate the original estimate and the calculated estimate, optionally with further information as described above, to an external system via a suitable data output interface 104 as described above. For example, the apparatus 100 may communicate these data to an external system each time an estimate was calculated. Alternatively, the apparatus may store the data and communicate the collected data for a plurality of meals to an external system. Consequently, the user, health care personal or others may analyse the data so as to arrive at recommendations as to how the user may improve the carbohydrate estimates and thus the insulin therapy.

It will be apprectaited that the apparatus 100 may further perform additional functions for assisting the user in an improved insulin administration. For example, the apparatus may keep track on the total daily carbohydrate intake and compare it with the recommended daily intake for a person of that size, activity level (if known) and age. The activity level may be estimated for instance from a heart rate monitor or an accelerometer in a dosing device such as a pen or a pump or a measurement device such as a CGM or stick based blood glucose monitor or other device carried by the user. If there is a large discrepancy between the total daily carbohydrate intake reported by the user and the recommended intake, the user may be advised to adjust the meal estimates. For example, for a single day a 15% discrepancy may be taken as threshold. Similarly, if the user departs from the expected daily carbohydrate intake by more than e.g. 5% over a week the user may be alerted and asked for changes in weight or activity level.

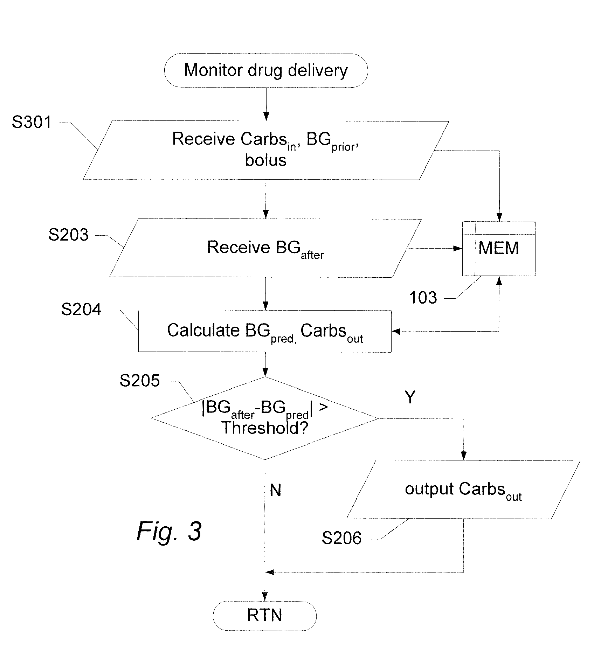

Fig. 3 shows a flow diagram of another example of a process for monitoring insulin administration. The example of fig. 3 is similar to the process of fig. 2, except that in the example of fig. 3, the process receives the bolus dose of insulin as a further input value rather than calculating the bolus from the other input values. Consequently, in this example, the process receives in initial step S301 a blood glucose measurement ($BG_{prior}$) indicative of the blood glucose level of the user prior to a meal intake, an estimate of the carbohydrate content ($Carbs_{in}$) of the meal to be consumed, and a dose size of an administered bolus insulin ('bolus'). For example, the apparatus 100 may receive the bolus dose size directly from a bolus calculator or from a drug delivery device, e.g. an insulin pen or an insulin pump, separate from the apparatus 100. Alternatively, the user may manually enter the bolus value into the apparatus 100, e.g. in situations where the user has determined the bolus value manually or with a device that has no data communication interface for directly communicating the determined bolus value. Accordingly, in the example of fig. 3, the process may not need to receive further input values useful for the bolus calculation. However, some of these values are also used as input to the physiological model used in step S204. The remaining steps S203 through S206 are performed as in the example of fig. 2.

Example of a Physiological model:

A physiological model was constructed by combining an insulin model by Berger [3], a meal model by Lehmann [4] and the minimal model by Bergman [5]. Hence, useful meal estimation feedback is possible based on a relative simple model. This model successfully models BG changes in the normoglycemic range. The compartment model includes six compartments as shown in fig. 4.

Four of the compartments may be described directly by differential equations. These are plasma insulin PI (402), insulin action IA (403), blood glucose BG (404) and gut glucose content $G_{gut}$ (406). Subcutaneous insulin level SC (401) and the stomach contents of glucose ST (405) can be derived, but are not, however, of main interest in the present context.

An example of a compartment model as shown in fig. 4 will now be described The relevant equations will be explained below with parameters described in Table 1.

Pharmacokinetic model of uptake of exogenous insulin [3]:

$$\frac{dPI(t)}{dt} = \frac{s \cdot t^{s-1} \cdot T_{50}^s}{(T_{50}^s + t^s)^2} \cdot dose - k \cdot PI(t) \qquad (E1)$$

$$T_{50} = a \cdot dose + b \text{ ,}$$

where s is the observed sigmoidicity in the time course of absorption, *t* is the time in minutes. $T_{50}$ is the time interval to permit 50% of the injected insulin dose to be absorbed. The parameter dose is the injected dose of insulin. The parameter k is the first-order elimination constant. The parameter *a* is the dose dependency of absorption time. The parameter b is the offset of insulin absorption time.

Pharmacodynamic model for glucose disappearance [5]:

$$\frac{dIA(t)}{dt} = p_3 \cdot PI(t) - p_2 \cdot IA(t) \text{ ,} \qquad (E2)$$

where $p_2$ is the rate of insulin action, and $p_3$ transendothelial insulin transport.

$$\frac{dBG(t)}{dt} = G_{in}(t) + p_1 \cdot G_b \cdot V_G - p_1 \cdot BG(t) - IA(t) \cdot BG(t) \text{ ,} \qquad (E3)$$

where $G_{in}$ is the glucose absorbed from the gut to the blood, $p_1$ is the effect of glucose on own utilization, $G_b$ is the Basal glucose absorption and VG is the distribution volume of glucose.

$$BGC(t) = \frac{BG(t)}{V_G},$$

where BGC is the blood glucose concentration.

Model of meal ingestion [4]:

$$\frac{d(G_{gut})}{dt} = G_{empt} - k_{gabs} \cdot G_{gut}, \qquad\qquad\qquad (E4)$$

Where $G_{empt}$ is the rate of gastric emptying and $k_{gabs}$ is the rate constant of glucose absorption from the gut to the blood.

$$G_{in}(t) = k_{gabs} \cdot G_{gut}$$

These can be calculated by the following algorithm:

$$T\max_{ge} = \frac{Ch - V\max_{ge} \cdot (Tasc_{ge} + Tdes_{ge})}{V\max_{ge}}$$

Where $T_{max\ ge}$ is the time period where glucose absorption from the gut is maximal, Ch is the carbohydrate contents of the meal, $V_{max\ ge}$ is the maximal rate of gastric emptying and $T_{asc\ ge}$ and $T_{des\ ge}$ is the relative length of the ascending and descending branches of the gastric emptying curve.

$$Ch_{crit} = 0.5 \cdot (V\max_{ge} \cdot (Tasc_{ge} + Tdes_{ge})),$$

where Gh$_{crit}$ is the meal size which does not cause the maximum rate of gastric emptying to be reached.

$$Tasc_{ge} = Tdes_{ge} = \frac{2 \cdot Ch}{V\max_{ge}}, \text{ if } Ch <= Ch_{crit}$$

$$G_{empt} = \frac{t \cdot V\max_{ge}}{Tasc_{ge}}, \text{ if } t < Tasc_{ge},$$

where t is the time from intake of the meal.

$$G_{empt} = V\max_{ge}, \;\; \text{if} \;\; Tasc_{ge} < t \leq Tasc_{ge} + T\max_{ge}$$

$$G_{empt} = V\max_{ge} - \frac{V\max_{ge} \cdot (t - Tdes_{ge} - T\max_{ge})}{Tdes_{ge}},$$

if $Tasc_{ge} + T\max_{ge} \leq t < Tasc_{ge} + Tdes_{ge} + T\max_{ge}$
$G_{empt} = 0$ elsewhere

All included parameters were adjusted according to an average person with type 1 diabetes. For the purpose of the present example, parameters specified in a number of published papers [7]-[9] were used:

Table 1. Description of the model parameters and the values used in the the present example.

| Parameter | Description | Value |
|---|---|---|
| $a$ | Dose dependency of absorption time [9] | $0.42*10^{-3}$ (min/pmol) |
| $b$ | Offset of insulin absorption time [9] | 0 (min) |
| s | The observed sigmoidicity in the time course of absorption [9] | 2.1 |
| $k$ | The first-order elimination constant [9] | 0.016 |
| $p_1$ | Effect of glucose on own utilization [7] | 0 |
| $p_2$ | Rate of insulin action [7] | $2.5*10^{-2}$ (min$^{-1}$) |
| $p_3$ | Transendothelial insulin transport [7] | $13*10^{-6}$ (min$^{-2}$ /$\mu$U/ml) |
| $V_G$ | Distribution volume of glucose [7] | 12 (L) |
| $V_I$ | Distribution volume of insulin [9] | 13.5 (L) |
| $G_{bx}$ | Basal glucose absorption | Equal to basal ins. level |
| $I_b$ | Basal insulin level [7] | 90 (pmol/L) |
| $V_{max\,ge}$ | Maximal rate of gastric emptying [4] | 2 (mmol/min) |

In order to maintain a constant blood glucose level, a constant basal glucose absorption level, $G_{bx}$, was added to equation (E3) to be balanced with the basal insulin level, $I_b$, as found in [7].

This model takes in information about carbohydrate contents of meals, amount, type and time for insulin injection or infusions and blood glucose values. The user's physiological values (blood glucose, gut contents, plasma insulin) are then simulated by iterating the differential equations of the models one time step forward until new values are entered.

The above model has been used to predict a blood glucose value and then adjust the meal size to match the measured BG value. In the example, a user has an intake of 50 grams of carbohydrate at time 0, however only reports 40 grams. He injects 12 units of insulin aspart at time 0.

Fig. 5 shows the resulting simulated BG. The initial simulated BG is shown as dashed line 501. At time $t$=250 min. a measurement of the user's actual BG was performed indicated by cross 502. The algorithm detects a difference between the simulated BG value 503 at time $t$=250 min. and the measured BG value 502. Responsive to this difference, the algorithm adjusts the model input value indicative of the carbohydrate content of the latest meal in order to match the BG simulated curve to the measured BG. The resulting new simulated BG curve is shown as solid line 504. The adjusted carbohydrate content of the meal that gives raise to simulated curve 504 may then reported back to the user as guidance for future meal estimates.

Although some embodiments have been described and shown in detail, the invention is not restricted to them, but may also be embodied in other ways within the scope of the subject matter defined in the following claims.

For example, the invention has mainly been described in connection with insulin treatment of diabetes and the estimation of the carbohydrate content of meals. It will be appreciated, however, that additional or alternative nutritional parameters, e.g. the fat content, protein content, vitamin content, etc., may be relevant in connection with other types of medication and/or other types of physiological parameters.

For example, the process as described herein may be modified such that it also can be used when two measured blood glucose levels at different times are used as input with a certain time between measurements, but without any meal estimate or bolus dose size as input. In such a case, if the predicted blood glucose level deviates from the actually measured later BG by a level larger than a predetermined threshold, the model may determine a corresponding carbohydrate content of a hypothetical meal between the two measurements. The user may then be informed of the resulting estimated carbohydrate content and asked about the possibility of having had a meal or snack without a corresponding insulin bolus. When using the physiological model a higher accuracy is achieved when having a more correct starting point both regarding the starting blood glucose level and its inner variables.

Embodiments of the method described herein can be implemented by means of hardware comprising several distinct elements, and/or at least in part by means of a suitably programmed microprocessor. In the apparatus claims enumerating several means, several of these means can be embodied by one and the same element, component or item of hardware. The mere fact that certain measures are recited in mutually different dependent claims or described in different embodiments does not indicate that a combination of these measures cannot be used to advantage.

It should be emphasized that the term "comprises/comprising" when used in this specification is taken to specify the presence of stated features, integers, steps or components but does not preclude the presence or addition of one or more other features, integers, steps, components or groups thereof.

References:

[1] Graff MR, Gross TM, Juth SE, Charlson J: How well are individuals on intensive insulin therapy counting carbohydrates? Diabetes Res Clin Pract 50:S238-2000,

[2] Alemzadeh R, Goldberg T, Fort P, Recker B, Lifshitz F: Reported dietary intakes of patients with insulin-dependent diabetes mellitus: limitations of dietary recall. Nutrition 8:87-93, 1992

[3] Berger M, Rodbard D: Computer simulation of plasma insulin and glucose dynamics after subcutaneous insulin injection. Diabetes care 12:725-736, 1989

[4] Lehmann ED, Deutsch T: A physiological model of glucose-insulin interaction in type 1 diabetes mellitus. J Biomed Eng 14:235-242, 1992

[5] Bergman RN, Ider YZ, Bowden CR, Cobelli C: Quantitative Estimation of Insulin Sensitivity. Am J Physiol 236: E667-E677, 1979

[6] Bergman, R. N. The minimal model: yesterday, today and tomorrow, in: The Minimal Model Approach and Determination of Glucose Tolerance. 3-50. 1997. LSU Press.

[7] Furler SM, Kraegen EW, Smallwood RH, Chisholm DJ: Blood glucose control by intermittent loop closure in the basal mode: computer simulation studies with a diabetic model. Diabetes care 8:553-561, 1985

[8] Vicini P, Caumo A, Cobelli C: Glucose effectiveness and insulin sensitivity from the minimal models: consequences of undermodeling assessed by Monte Carlo simulation. IEEE Trans Biomed Eng 46:130-137, 1999

[9] Østerberg O, Erichsen L, Ingwersen SH, Plum A, Poulsen HE, Vicini P: Pharmacokinetic and pharmacodynamic properties of insulin aspart and human insulin. J Pharmacokinet Pharmacodyn 30:221-235, 2003

**Claims**

1.  An apparatus for monitoring administration of a medication to a user, the apparatus comprising processing means adapted to:

    - obtain input values indicative of at least a first and a second measurement of a physiological parameter of the user, and of at least a dose of medication administered to the user;
    - determine, from at least the obtained input values, an estimate of a nutritional parameter of a meal consumed by the user between the first and the second measurement;
    - generate an output indicative of the determined estimate.

2.  An apparatus according to claim 1, wherein the medication includes a medicament for affecting at least the physiological parameter of the user.

3. An apparatus according to any one of claims 1 though 2, wherein the medication includes a medicament for the treatment of diabetes, and wherein the physiological parameter is a glucose level.

4. An apparatus according to any one of claims 1 though 3, further comprising an output device for generating a user-detectable output indicative of the determined estimate.

5. An apparatus according to any one of claims 1 through 4, wherein the processing means is further adapted to receive an input value indicative of a preliminary estimate of the nutritional parameter or a value derived from the preliminary estimate of the nutritional parameter.

6. An apparatus according to claim 5, wherein the processing means is further adapted to generate, from the received input values an output indicative of a deviation between the determined estimate and the received preliminary estimate of the nutritional parameter.

7. An apparatus according to claim 5 or 6, wherein the processing means is further adapted to calculate deviations between respective determined estimates and respective received preliminary estimates corresponding to a plurality of meal intakes; and to determine an overall deviation from the calculated deviations.

8. An apparatus according to any one of claims 1 through 7, wherein the processing means is adapted to determine the estimate of the nutritional parameter of the meal from the obtained input values and from a physiological model.

9. An apparatus according to claim 8, wherein the physiological model is a user-specific model.

10. An apparatus according to claim 8 or 9, wherein the physiological model is a physiological prediction model for predicting a value of the physiological parameter after the meal intake from at least a value of the physiological parameter before the meal intake, from the dose of medication administered to the user, and from the estimated nutritional parameter of the meal.

11. An apparatus according to claim 10, wherein the processing means is adapted to determine the estimate of the nutritional parameter as a value of the nutritional parameter for which a difference between the predicted value of the physiological parameter and a measured value of the physiological parameter after the meal intake is smaller than a threshold.

12. An apparatus according to any one of claims 1 through 11, wherein the nutritional parameter is a carbohydrate content.

13. An apparatus according to any one of claims 1 through 12, wherein the processing means is further adapted to calculate a dose or a series of doses of medication based on at least the obtained input value of the physiological parameter of the user measured prior to intake of the meal by the user.

14. An apparatus according to any one of claims 8 through 9, wherein the apparatus is adapted to cooperate with means for measuring a glucose level of the user, and wherein the apparatus comprises an input device for obtaining the result of the glucose measurement from the means for measuring a glucose level.

15. A method of monitoring administration of a medication to a user, the method comprising:

- obtaining input values indicative of at least a first and a second measurement of a physiological parameter of the user, and of at least a dose of medication administered to the user;
- determining, from at least the obtained input values, an estimate of a nutritional parameter of a meal consumed by the user between the first and the second measurement;
- generating an output indicative of the determined estimate.

Fig. 1

Fig. 2

Monitor drug delivery

S301 — Receive Carbs$_{in}$, BG$_{prior}$, bolus

S203 — Receive BG$_{after}$

MEM

103

S204 — Calculate BG$_{pred}$, Carbs$_{out}$

S205 — $|BG_{after}-BG_{pred}| >$ Threshold?

Y

N

output Carbs$_{out}$

S206

*Fig. 3*

RTN

*Fig. 4*

*Fig. 5*

**European Patent Office**

**PARTIAL EUROPEAN SEARCH REPORT**
which under Rule 63 of the European Patent Convention shall be considered, for the purposes of subsequent proceedings, as the European search report

Application Number

EP 07 11 3689

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 97/28737 A (NOKIA MOBILE PHONES LTD [FI]; HEINONEN PEKKA [FI]; OKKONEN HARRI [FI]) 14 August 1997 (1997-08-14)<br>* abstract *<br>* page 2, line 12 - line 28 *<br>* page 3, line 25 - line 35 *<br>* page 11 - page 12 *<br>* figure 4 *<br>----- | 1-14 | INV.<br>G06F19/00<br>A61B5/00<br>A61M5/172<br>G01N33/487 |
| Y<br>A | WO 01/91633 A (INSTRUMENTATION METRICS INC [US]) 6 December 2001 (2001-12-06)<br>* the whole document *<br>* page 11, line 9 - line 29 *<br>----- | 1-12,14<br>1-14 | |
| Y<br>A | EP 1 571 582 A (BECTON DICKINSON CO [US]) 7 September 2005 (2005-09-07)<br>* the whole document *<br>* paragraphs [0009], [0012], [0013], [0036], [0039] - [0041], [0047] *<br>----- | 13<br>1-14 | |
| A | WO 03/023682 A (UNIVATION LTD [GB]; BUTLER RICHARD [GB]) 20 March 2003 (2003-03-20)<br>* the whole document *<br>-----<br>-/-- | 1-14 | TECHNICAL FIELDS SEARCHED (IPC)<br><br>A61B<br>A61M<br>G01N<br>G06F<br>A61J |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 29 November 2007 | Petersch, Bernhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

EP 2 023 256 A1

European Patent
Office

**PARTIAL EUROPEAN SEARCH REPORT**

Application Number

EP 07 11 3689

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| A | WO 2005/081171 A (MATHEWS EDWARD HENRY [ZA]) 1 September 2005 (2005-09-01)<br>* the whole document *<br>----- | 1-14 | |
| | | | **TECHNICAL FIELDS SEARCHED (IPC)** |

EPO FORM 1503 03.82 (P04C10)

21

Claim(s) searched completely:
        1-14

Claim(s) not searched:
        15

Reason for the limitation of the search (non-patentable invention(s)):

In the light of page 6, lines 20-28 of the description, claim 15 refers
to a method of monitoring administration of a medication to a user, the
corresponding apparatus being connected to an injection device or
infusion device (such as an infusion pump) for delivery of medication and
claim 15 thus constitutes a method for treatment of the human body by
therapy (Art.53(c) EPC).

**ANNEX TO THE EUROPEAN SEARCH REPORT**
**ON EUROPEAN PATENT APPLICATION NO.**                EP 07 11 3689

This annex lists the patent family members relating to the patent documents cited in the above-mentioned European search report.
The members are as contained in the European Patent Office EDP file on
The European Patent Office is in no way liable for these particulars which are merely given for the purpose of information.

29-11-2007

| Patent document cited in search report | | Publication date | Patent family member(s) | | Publication date |
|---|---|---|---|---|---|
| WO 9728737 | A | 14-08-1997 | AU | 1726797 A | 28-08-1997 |
| | | | DE | 69707266 D1 | 15-11-2001 |
| | | | DE | 69707266 T2 | 04-07-2002 |
| | | | DK | 883371 T3 | 28-01-2002 |
| | | | EP | 0883371 A1 | 16-12-1998 |
| | | | ES | 2163734 T3 | 01-02-2002 |
| | | | FI | 960637 A | 13-08-1997 |
| | | | JP | 3735660 B2 | 18-01-2006 |
| | | | JP | 2000503556 T | 28-03-2000 |
| | | | US | 5840020 A | 24-11-1998 |
| WO 0191633 | A | 06-12-2001 | AU | 5745901 A | 11-12-2001 |
| | | | EP | 1292213 A1 | 19-03-2003 |
| | | | JP | 2004506610 T | 04-03-2004 |
| | | | US | 2002132279 A1 | 19-09-2002 |
| EP 1571582 | A | 07-09-2005 | CA | 2498682 A1 | 01-09-2005 |
| | | | JP | 2005315855 A | 10-11-2005 |
| | | | US | 2005192494 A1 | 01-09-2005 |
| WO 03023682 | A | 20-03-2003 | CA | 2459673 A1 | 20-03-2003 |
| | | | EP | 1428165 A2 | 16-06-2004 |
| | | | GB | 2381907 A | 14-05-2003 |
| | | | JP | 2005502406 T | 27-01-2005 |
| | | | US | 2005071141 A1 | 31-03-2005 |
| WO 2005081171 | A | 01-09-2005 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2004180810 A1
- US 2005065760 A
- US 2003114836 A
- US 2006272652 A
- WO 0010628 A
- WO 0018293 A


**Non-patent literature cited in the description**

- **KILDEGAARD J ; RANDLØV J ; POULSEN JU ; HEJLESEN OK.** The impact of non-model related variability on blood glucose prediction. *Diabetes Technology and Therapeutics,* 2007
- **GRAFF MR ; GROSS TM ; JUTH SE ; CHARLSON J.** How well are individuals on intensive insulin therapy counting carbohydrates?. *Diabetes Res Clin Pract,* vol. 50, 238-2000
- **ALEMZADEH R ; GOLDBERG T ; FORT P ; RECKER B ; LIFSHITZ F.** Reported dietary intakes of patients with insulin-dependent diabetes mellitus: limitations of dietary recall. *Nutrition,* 1992, vol. 8, 87-93
- **BERGER M ; RODBARD D.** Computer simulation of plasma insulin and glucose dynamics after subcutaneous insulin injection. *Diabetes care,* 1989, vol. 12, 725-736
- **LEHMANN ED ; DEUTSCH T.** A physiological model of glucose-insulin interaction in type 1 diabetes mellitus. *J Biomed Eng,* 1992, vol. 14, 235-242
- **BERGMAN RN ; IDER YZ ; BOWDEN CR ; COBELLI C.** Quantitative Estimation of Insulin Sensitivity. *Am J Physiol,* 1979, vol. 236, E667-E677
- The minimal model: yesterday, today and tomorrow. **BERGMAN, R. N.** The Minimal Model Approach and Determination of Glucose Tolerance. LSU Press, 1997, 3-50
- **FURLER SM ; KRAEGEN EW ; SMALLWOOD RH ; CHISHOLM DJ.** Blood glucose control by intermittent loop closure in the basal mode: computer simulation studies with a diabetic model. *Diabetes care,* 1985, vol. 8, 553-561
- **VICINI P ; CAUMO A ; COBELLI C.** Glucose effectiveness and insulin sensitivity from the minimal models: consequences of undermodeling assessed by Monte Carlo simulation. *IEEE Trans Biomed Eng,* 1999, vol. 46, 130-137
- **ØSTERBERG O ; ERICHSEN L ; INGWERSEN SH ; PLUM A ; POULSEN HE ; VICINI P.** Pharmacokinetic and pharmacodynamic properties of insulin aspart and human insulin. *J Pharmacokinet Pharmacodyn,* 2003, vol. 30, 221-235